# EUROPÄISCHE PATENTANMELDUNG

(11) **EP 4 299 002 A1**
(43) Veröffentlichungstag der Anmeldung: **03.01.2024**
(21) Anmeldenummer: 22181714.1
(22) Anmeldetag: 29.06.2022
(51) Int. Cl.: A61B 5/055, G01R 33/20

(54) **VORRICHTUNG UND VERFAHREN ZUR SICHEREN KOMMUNIKATION ZWISCHEN EINEM PERIPHERIEGERÄT UND EINEM MEDIZINGERÄT**

(71) Anmelder: Siemens Healthcare GmbH, 91052 Erlangen (DE)
(72) Erfinder: Biber, Stephan, 91056 Erlangen (DE)
(74) Vertreter: Siemens Healthineers Patent Attorneys

(57) **Zusammenfassung**

Die Erfindung betrifft ein Peripheriegerät für ein Medizingerät, vorzugsweise einen Magnetresonanztomographen, ein System aus dem Peripheriegerät und dem Medizingerät und ein Verfahren zum Betrieb des Peripheriegeräts mit dem Medizingerät. Das Peripheriegerät weist eine kryptographische Einheit auf und das Peripheriegerät und das Medizingerät kommunizieren mit Hilfe der kryprographischen Einheit.

## Beschreibung

Die Erfindung betrifft ein Peripheriegerät für ein Medizingerät, sowie ein System aus einem Medizingerät und ein Verfahren für einen gesicherten Betrieb des Medizingeräts mit dem Peripheriegerät.

Eine Vielzahl von Medizingeräten benötigt zum Betrieb Peripheriegeräte. Dies können beispielsweise Sensoren und Aktoren sein. Bildgebenden Modalitäten verwenden beispielsweise Sensoren, um Bewegungen oder physiologische Daten des Patienten zu erfassen und damit den Patienten zu überwachen oder die erfassten Werte bei der Bildgebung zu berücksichtigen, beispielsweise die Bilderfassung damit zu synchronisieren. Auch sind Aktoren in Verwendung, um beispielsweise Kontrastmittel an den Patienten abzugeben. Bei Magnetresonanztomographen werden darüber hinaus die für die Bildgebung erforderlichen Magnetresonanzsignale mit sogenannten Lokalspulen aufgenommen, die in unterschiedlichen Varianten für die zu untersuchenden Körperteile existieren. Die Lokalspulen sind austauschbare Peripheriegeräte, die über Steckverbindungen oder auch drahtlos mit dem Magnetresonanztomographen verbunden sind.

Die Sicherheit des Patienten ist bei Medizingeräten von zentraler Bedeutung. Einige Peripheriegeräte sind nur für den einmaligen Gebrauch vorgesehen. Hygieneanforderungen erfordern, dass zumindest das gleiche Peripheriegerät nicht unmittelbar hintereinander an unterschiedlichen Patienten verwendet wird, sondern nach entsprechenden Vorgaben sterilisiert werden. Auch kann durch versehentliches Vertauschen oder durch Verwendung vermeintlich kompatibler Peripheriegeräte eine Fehlfunktion verursacht werden, die den Patienten gefährdet. Nicht zuletzt muss bei einigen Peripheriegeräten eine turnusgemäße und korrekte Wartung sichergestellt werden.

Für Magnetresonanztomographen ist es bekannt, einen Parameterspeicher einer Lokalspule über einen I2C-Bus auszulesen und im Magnetresonanztomographen dementsprechende Einstellungen vorzunehmen.

Es ist daher eine Aufgabe der vorliegenden Erfindung, die Verwendung eines Medizingeräts mit einem Peripheriegerät sicherer zu machen.

Die Aufgabe wird durch ein erfindungsgemäßes Peripheriegerät nach Anspruch 1, durch ein erfindungsgemäßes System aus einem Medizingerät und einem erfindungsgemäßen Peripheriegerät nach Anspruch 7 und einem Verfahren zum Betrieb derselben nach Anspruch 8 gelöst.

Das erfindungsgemäße Peripheriegerät ist für ein Medizingerät vorgesehen. Mit Medizingerät wird eine Vorrichtung bezeichnet, die zur Behandlung oder Untersuchung eines Patienten genutzt werden. Insbesondere werden darunter auch bildgebende Modalitäten wie Ultraschallscanner, Röntgengeräte oder Magnetresonanztomographen verstanden.

Als Peripheriegerät werden dabei Vorrichtungen angesehen, die lösbar mit dem Medizingerät zur Übertragung einer Information bzw. Anweisung verbindbar sind und zu dessen ordnungsgemäßer Anwendung zumindest in einem Anwendungsfall erforderlich sind. Dies können beispielsweise Sensoren zur Erfassung von Signalen für eine Bildgebung, von physiologischen Aktivitäten des Patienten oder auch Aktuatoren zur Beeinflussung des Patienten sein. Die Verbindung kann kabelgebunden oder auch drahtlos sein.

Das Peripheriegerät weist eine kryptographische Einheit für eine Kommunikation mit dem Medizingerät auf. Als kryptographische Einheit wird eine Einheit angesehen, die mittels kryptographischer Methoden eine Information in ihrem Inneren und/oder auf der Verbindung mit dem Medizingerät vor Zugriff oder Veränderung durch unbefugte Dritte sichert. Insbesondere wird als kryptographische Methode auch ein Challenge-Response-Verfahren angesehen, mit dem sich das Peripheriegerät mittels einer Information in der kryptographischen Einheit gegenüber dem Medizingerät authentifizieren kann.

Die kryptographische Einheit kann als Software in einem Prozessor des Peripheriegeräts vorgesehen sein. Vorzugsweise weist der Prozessor dabei Hardwaremerkmale auf, um die kryptographischen Informationen oder Vorgänge vor einem unberechtigten Zugriff von außen zu schützen. Es sind aber auch dedizierte kryptographische Bausteine denkbar, die kryprographische Informationen oder Verarbeitungsschritte vor einem Zugang bzw. Ausforschen von außen schützen.

Auf vorteilhafte Weise ermöglicht die kryptographische Einheit eine sichere Kommunikation und/oder Authentifizierung des Peripheriegeräts.

Das erfindungsgemäße Verfahren ist zum Betrieb eines Medizingeräts mit einem erfindungsgemäßen Peripheriegerät vorgesehen.

In einem Schritt des erfindungsgemäßen Verfahrens wird das Peripheriegerät durch das Medizingerät mittels einer Kommunikation mit der kryptografischen Einheit authentifiziert.

Beispielsweise kann die kryptographische Einheit eine Verschlüsselung der Verbindung bereitstellen. Über diese Verbindung kann die kryptographische Einheit eine Kennung mit dem Medizingerät austauschen. Die Kennung kann beispielsweise in der kryptographischen Einheit abgelegt sein, möglich ist aber auch ein Speicher in dem Peripheriegerät oder der Prozessor. Die Authentifizierung erfolgt dann durch das Medizingerät. Wenn die Kennung vorbestimmten Kriterien erfüllt, beispielsweise einem bestimmten Wert hat oder einem vorbestimmten Prüfalgorithmus genügt, ist die Authentifizierung positiv bzw. erfolgreich.

Ein sicheres Verfahren, das auch ohne Verschlüsselung der Übertragung auskommt, ist ein Challenge-Response Verfahren. Ein geheimer Schlüssel ist in der kryptographischen Einheit abgelegt und ein öffentlicher Schlüssel des Peripheriegeräts in dem Medizingerät. Das Medizingerät schickt beispielsweise eine Nachricht an die kryptographische Einheit. Vorzugsweise ist die Nachricht mit dem öffentlichen Schlüssel verschlüsselt. Die kryptographische Einheit entschlüsselt die Nachricht mit dem geheimen Schlüssel und sendet das Ergebnis zurück. Wenn das Ergebnis mit der ursprünglichen Nachricht übereinstimmt, ist das Peripheriegerät erfolgreich authentifiziert. Es sind aber auch beliebige andere Varianten einer Challenge-Response Authentifizierung denkbar, bei denen zum Beispiel die Rollen von öffentlichem und geheimen Schlüssel vertauscht sein können oder von Ver- und Entschlüsselung etc. Insbesondere sind auch Varianten denkbar, in denen mit kryptographischen Mitteln ein Hash der Nachricht gebildet wird, um diese zu authentifizieren. Dieser kryptographische Hash kann auch unverschlüsselt übertragen werden bzw. auch die Nachricht selbst, sofern die Nachricht selbst kein zu schützendes Geheimnis aufweist, sondern nur die Quelle authentifiziert werden muss.

Die Authentifizierung bzw. die dabei verwendete Nachricht kann auch veränderlich sein, d.h. von verschiedenen Parametern wie Alter oder bisheriger Nutzung abhängig sein. Beispiele dafür sind zu den Unteransprüchen erläutert.

In einem weiteren Schritt gibt das Medizingerät eine Nutzung des Peripheriegeräts frei, wenn die Authentifizierung erfolgreich war. Beispielsweise wird eine Eingabe des Nutzers erst von einer Steuerung des Medizingeräts ausgeführt, wenn die Authentifizierung erfolgreich war.

Das erfindungsgemäße Verfahren teilt die Vorteile des erfindungsgemäßen Peripheriegeräts.

Weitere vorteilhafte Ausführungsformen sind zu den Unteransprüchen angegeben.

In einer denkbaren Ausführungsform ist das Medizingerät ein Magnetresonanztomograph. Ein Magnetresonanztomograph nutzt häufig Lokalspulen als Peripheriegeräte zum Empfang von Magnetresonanzsignalen, die für unterschiedliche Untersuchungen von unterschiedlichen Körperteilen getauscht werden müssen. Um die Sicherheit des Patienten zu gewährleisten, müssen dabei die Lokalspulen eindeutig identifiziert werden. Denkbar sind aber auch andere Peripheriegeräte wie Sensoren für Körperfunktionen wie Herzschlag oder Atembewegung. Auch Aktoren zur Abgabe von Kontrastmitteln sind denkbar.

Auf vorteilhafte Weise stellt das erfindungsgemäße Peripheriegerät eine sichere Nutzung des Magnetresonanztomographen bereit.

In einer möglichen Ausführungsform ist die kryptografische Einheit ausgelegt, die Kommunikation mit dem Medizingerät zu verschlüsseln. Denkbar ist beispielsweise, dass die kryptographische Einheit eine Verschlüsselung in Software oder durch eine Hardware vornimmt. Denkbar ist beispielsweise eine Verschlüsselung nach RSA, AES oder einem anderen Standard.

Auf vorteilhafte Weise sorgt die Verschlüsselung dafür, dass eine Kennung bei der Übertragung nicht auf dem Übertragungsweg erfasst und unerlaubt für unzulässige Peripheriegeräte genutzt oder verändert werden kann.

In einer denkbaren Ausführungsform des erfindungsgemäßen Peripheriegeräts nach einem der vorhergehenden Ansprüche ist die kryptographische Einheit ausgelegt, ein kryptographisches Geheimnis zu speichern. Beispielsweise kann die kryptographische Einheit ausgelegt sein, einen öffentlichen oder geheimen Schlüssel zu speichern. Insbesondere bei einem geheimen Schlüssel ist die kryptographische Einheit auch geeignet, einen Zugriff zum Auslesen des geheimen Schlüssels zu verhindern. Das Magnetresonanzgerät ist vorzugsweise ausgelegt in der Kommunikation mit dem Medizingerät eine Challenge-Response zu beantworten, also die bereits beschriebenen Schritte des Verfahrens auszuführen.

Auf vorteilhafte Weise ermöglicht das Speichern als ein kryptographisch geschütztes Geheimnis zuverlässig eine Manipulation oder Bekanntwerden der Schlüssel zu verhindern.

In einer möglichen Ausführungsform des erfindungsgemäßen Peripheriegeräts weist die kryptographische Einheit einen geschützten Zähler auf. Die kryptographische Einheit ist geeignet, den geschützten Zähler auf vorbestimmte Weise und sicher gegen nicht autorisierte Manipulationen zu verändern, beispielsweise zu inkrementieren oder dekrementieren.

Auf vorteilhafte Weise kann so beispielsweise eine Nutzung auf sichere Weise limitiert werden oder ein manipulationssicherer Servicezyklus implementiert werden.

Das erfindungsgemäße System weist ein erfindungsgemäßes Peripheriegerät und ein Medizingerät auf. Das Peripheriegerät und das Medizingerät sind ausgelegt, in Zusammenwirken eine Bilderfassung, Untersuchung oder Behandlung an einem Patienten zu unterstützen. Beispielsweise kann das Peripheriegerät ein Sensor für eine Bilderfassung mit dem bildgebenden Medizingerät sein, wie beispielsweise eine Lokalspule bei einem Magnetresonanztomographen, ein Röntgendetektor bei einem Röntgengerät oder ein Ultraschallkopf bei einem Ultraschallgerät. Möglich ist auch ein Aktuator, der ein Kontrastmittel im Rahmen der bildgebenden Untersuchung an den Patienten abgibt. Es sind aber auch Sensoren oder Aktuatoren bei anderen Medizingeräten denkbar wie beispielsweise Patientenmonitore oder Dosiergeräte für Medikamente.

Das Medizingerät ist dabei ausgelegt, mit der kryptographischen Einheit zu kommunizieren. Die Kommunikation zwischen der kryptographischen Einheit des Peripheriegeräts und dem Medizingerät kann dabei auf unterschiedlichen physischen Signalverbindungen erfolgen wie elektrische oder optische Kabelverbindungen oder drahtlose Verbindungen mit Radiowellen, beispielsweise Frequenzen größer als 100 MHz, 1 GHz oder 10 GHz, vorzugsweise in einem ISM-Band, oder optischen Wellen, beispielsweise im nahen Infrarotbereich oder sichtbaren Licht. Als Kommunikation wird es im Sinne der Erfindung angesehen, wenn die kryptographische Einheit und das Medizingerät Zugriff auf den Inhalt von ausgetauschten Nachrichten haben, sei es, dass die Nachrichten selbst unverschlüsselt sind oder die kryptographische Einheit und das Medizingerät in der Lage sind, diese zu entschlüsseln.

Selbst ohne Verschlüsselung können die kryptographische Einheit beispielsweise mit einem Challenge-Response-Verfahren eine sichere Authentifizierung durchführen. Mit Verschlüsselung ist es auch möglich, komplexere Interaktionen zwischen Peripheriegerät und Medizingerät auszuführen, einschließlich der gesicherten Übertragung der erfassten Daten für eine Bildgebung.

Auf vorteilhafte Weise ermöglicht die kryptographisch gesicherte Kommunikation zwischen Peripheriegerät und Medizingerät gesicherte Funktionen wie Authentifizierung, Datenaustausch oder Datenmanipulation.

In einer möglichen Ausführungsform des erfindungsgemäßen Verfahrens weist die kryptographische Einheit einen kryptographisch geschützten Zähler auf. Als Zähler wird dabei jedes Datenobjekt angesehen, mit dem sich eine Anzahl an vorbestimmten Vorgängen erfassen lässt. Denkbar ist beispielsweise ein Datum vom Typ Integer, das mit jedem Vorgang inkrementiert oder dekrementiert wird. Denkbar ist auch ein Bit als Zähler, wenn nur ein einzelner Vorgang erfasst werden soll. Grundsätzlich sind aber auch andere Datentypen denkbar wie beispielsweise Fließkommazahlen oder Strings.

Als kryptographisch gesichert wird ein Zähler angesehen, der entweder gar nicht manipuliert werden kann, d.h. nur durch den zu zählenden Vorgang in der vorbestimmten Richtung geändert wird, nicht aber auf einen beliebigen Wert gesetzt oder zurückgesetzt werden kann. Als kryptographisch gesichert wird es auch angesehen, wenn die Manipulation nur für einen dafür authentifizierten Nutzer und kryptographisch gesichert erfolgen kann, beispielsweise über eine verschlüsselte Verbindung.

Das Authentifizieren des Peripheriegeräts gegenüber dem Medizingerät erfolgt dabei in Abhängigkeit von einem vorbestimmten Zählerstand des kryptographisch geschützten Zählers erfolgt.

Beispielsweise kann mit dem Zähler eine Anzahl an Vorgängen erfasst werden. Bei dem Erreichen einer vorbestimmten Anzahl an Vorgängen wie z.B. Bilderfassungen kann es aus Sicherheitsgründen erforderlich sein, das Gerät auf eine korrekte Funktion zu überprüfen. Das Peripheriegerät authentifiziert sich bei Erreichen des vorbestimmten Zählerstandes nicht mehr bei dem Medizingerät bzw. wird von dem Medizingerät nicht mehr erfolgreich authentifiziert und verhindert so eine unsichere Nutzung. Servicepersonal kann sich nach erfolgter Prüfung bei dem Peripheriegerät authentifizieren, Zugriff auf den Zählererhalten und diesen zurücksetzen. Denkbar ist auch, dass die Anzahl der Nutzungsvorgänge grundsätzlich limitiert ist und das Peripheriegerät eine weitere Authentifizierung bei Erreichen des Limits endgültig ablehnt. Dies stellt auch bei Peripheriegeräten zur einmaligen Nutzung zuverlässig das notwendige Ersetzen sicher.

Das Medizingerät über eine kryptographisch gesicherte Verbindung oder das Peripheriegerät selbst ändert den Zähler vorzugsweise zu bestimmten Zeitpunkten während der Untersuchung. Bevorzugt ist das Herunterzählen des Counters und eine Verweigerung der Spulennutzung, falls der Counter 0 erreicht hat. Herunterzählen des Zählers kann beispielsweise beim Stecken der Lokalspule, nach Abschluss der Justagen, am Ende der Untersuchung erfolgen, wobei dies durch eine Kombination mehrerer Ereignisse erkannt werden kann, wie z.B. durch Stecken der Lokalspule, Messung und dann Bewegung des Patiententisches in Richtung der Beladeposition.

Um sicherzustellen, dass z.B. wegen einer Falschpositionierung, bei der eine Lokalspule neu gesteckt wird, nicht die Lokalspule als unbenutzbar gezählt wird, ist es auch möglich, dass auf dem System eine Zeitschrank vorgesehen ist:
Beim Anstecken des Peripheriegeräts wird der Zähler um 1 heruntergesetzt. Das Peripheriegerät ist aber danach noch z.B. für mindestens 10 min, 20 min, 60 min oder 180 min erfolgreich authentifizierbar und/oder benutzbar, auch wenn der Zähler bereits den vorbestimmten Grenzwert erreicht hat. Der Magnetresonanztomograph kann sich beispielsweise eine ID des EEPROMs oder eine ID des Peripheriegeräts merken, und für dieses Peripheriegerät für die genannte Zeit einen Betrieb zulassen, auch wenn der Zähler der Spule bereits den Grenzwert erreicht hat.

Auf vorteilhafte Weise sichert so die Zeitschranke einen zuverlässigen Betrieb und vermeidet unnötigen Verwurf.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Systems;
- Fig. 2: eine schematische Darstellung eines erfindungsgemäßen Peripheriegeräts mit einer kryptographischen Einheit;
- Fig. 3: eine schematische Darstellung eines kryptographischen Informationsaustauschs;
- Fig. 4: eine schematische Darstellung eines Ablaufplans einer Ausführungsform des erfindungsgemäßen Verfahrens.

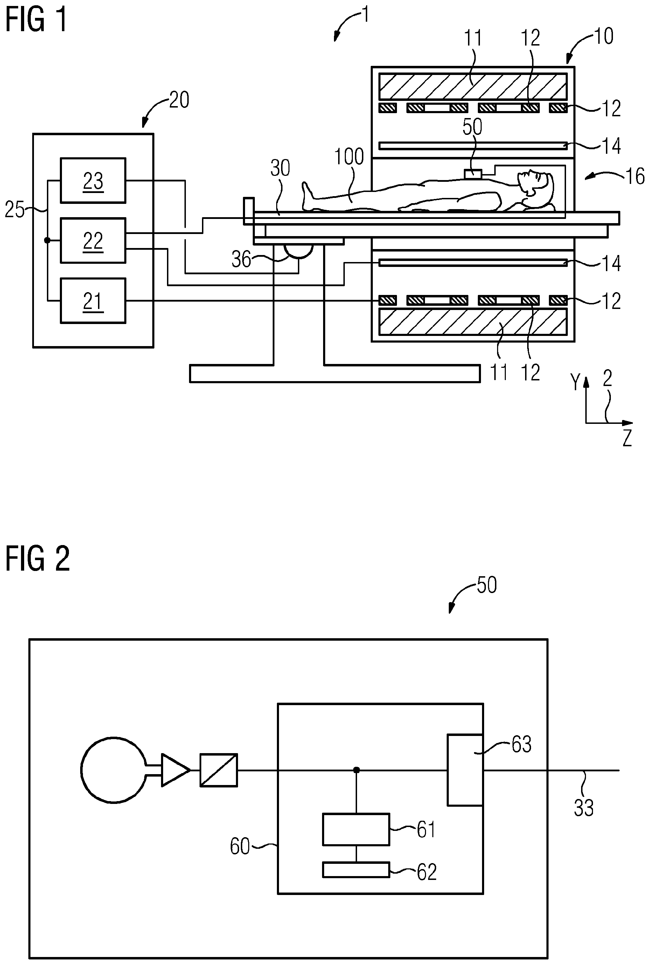

Fig. 1 zeigt eine schematische Darstellung einer beispielhaften Ausführungsform eines erfindungsgemäßen Systems aus einem Magnetresonanztomographen 1 als Medizingerät und mit einer Lokalspule 50 als Peripheriegerät.

Die Magneteinheit 10 weist einen Feldmagneten 11 auf, der ein statisches Magnetfeld B0 zur Ausrichtung von Kernspins von Proben bzw. Patienten 100 in einem Aufnahmebereich erzeugt. Der Aufnahmebereich ist in einem Patiententunnel 16 angeordnet, der sich in einer Längsrichtung 2 durch die Magneteinheit 10 erstreckt. Ein Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung 33 zugeführtes Hochfrequenzsignal in das Untersuchungsvolumen abzustrahlen und von dem Patient 100 emittierte Resonanzsignale zu empfangen und über eine Signalleitung abzugeben. Bevorzugter Weise wird aber die Körperspule 14 für das Aussenden des Hochfrequenzsignals und/oder das Empfangen durch Lokalspulen 50 ersetzt, die in dem Patiententunnel 16 nahe am Patient 100 angeordnet sind. Es ist aber auch denkbar, dass die Lokalspule 50 zum Senden und Empfangen ausgelegt ist und deshalb eine Körperspule 14 entfallen kann.

Eine Steuereinheit 20 versorgt die Magneteinheit 10 mit den verschiedenen Signalen für die Gradientenspulen 12 und die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus 25 untereinander verbunden.

Das von Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar ist aber auch ein Aussenden des Hochfrequenzsignals über eine oder mehrere Lokalspulen 50.

Die Lokalspule 50 empfängt dann vorzugsweise ein Magnetresonanzsignal aus dem Körper des Patienten 100, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet. Vorzugsweise wird dazu die Signalverbindung 33 genutzt, es ist aber beispielsweise auch eine drahtlose Übertragung denkbar.

Die Signalverbindung 33 zwischen der Lokalspule 50 und der Steuereinheit 20 ist von außen zugänglich und dadurch Angriffen wie abhören oder Simulation eines Partners, beispielsweise auch durch Fremdprodukte, gefährdet. Um die Kommunikation oder zumindest kritische Teile davon zu sichern, weist deshalb ein erfindungsgemäßes Peripheriegerät eine kryptographische Einheit 60 auf.

In Fig. 2 sind Details eines erfindungsgemäßen Peripheriegeräts, hier beispielhaft eine Lokalspule 50, mit der kryptographischen Einheit 60 dargestellt.

Die kryptographische Einheit 60 weist in der Ausführungsform der Fig. 2 eine Verschlüsselungseinheit 63 auf. Die Verschlüsselungseinheit 63 ist dazu ausgelegt, eine Kommunikation mit dem Medizingerät, in diesem Beispiel dem Magnetresonanztomographen 1 zu verschlüsseln. Als Verschlüsselungsverfahren können beispielsweise RSA oder AES zur Anwendung kommen. Die Verschlüsselung wird durch eine komplementäre Verschlüsselungseinheit in dem Medizingerät wieder entschlüsselt.

Es ist dabei denkbar, dass die Verschlüsselung auch Nutzdaten der Lokalspule 50 für die Bilderfassung umfasst. Ebenso ist es aber denkbar, dass die Verschlüsselung nur die zu schützende Kommunikation der kryptographischen Einheit 60 umfasst und Magnetresonanzdaten der Lokalspule 50 über eine eigene Leitung der Signalverbindung 33 übertragen werden oder mit den Daten der kryptographischen Einheit 60 gemultiplext werden. So kann eine Verschlüsselungseinheit 63 mit geringer Leistungsfähigkeit und vorteilhaft geringem Leistungsbedarf genutzt werden. Das Multiplexen kann auch in der kryptographischen Einheit 60 erfolgen.

Die kryptographische Einheit 60 weist weiterhin einen Prozessor auf, der ausgelegt ist, zu schützende kryptographische Prozesse auszuführen, auf die nicht von außen zugegriffen werden soll. Derartige kryptografische Prozesse können beispielsweise den Austausch bzw. das Aushandeln von Schlüsseln für die Verschlüsselung betreffen, das Bearbeiten einer Challenge-Response Anfrage und/oder das Berechnen von Hashtags.

Darüber hinaus kann die kryprographische Einheit 60 auch einen kryptographisch gesicherten Speicher 62 aufweisen, in dem ein nicht manipulierbares oder nur auf vorbestimmte Weise veränderliches Geheimnis bzw. Datum abgelegt werden kann. Dies kann beispielsweise ein Schlüssel für die Verschlüsselung, eine Authentifizierung oder auch ein manipulationssicherer Zähler sein.

Die kryptographische Einheit 60 weist vorzugsweise einen Prozessor 61 auf. Je nach Sicherheitsanforderung kann der Prozessor 61 auf verschiedene Weisen implementiert sein. Wird beispielsweise das Peripheriegerät bzw. dessen Inneres als sicher angesehen, kann der Prozessor der kryptographische Einheit 60 durch handelsübliche Prozessoren und externe Peripheriebausteine realisiert sein.

Eine höhere Sicherheitsstufe kann mit Software erreicht werden, die auf einem monolithischen Controllerbaustein als Prozessor 61 läuft, der durch geeignete Schutzmaßnahmen das Auslesen des internen Speichers verhindert oder gar die Daten intern verschlüsselt. Als höchste Stufe der Sicherheit existieren spezielle kryptographische Prozessoren, die auch interne Vorgänge verschleiern, um ein Auslesen auch mit aufwändigen Maßnahmen unmöglich zu machen.

Die Lokalspule 50 weist weiterhin die erforderlichen Einheiten zum Empfang eines Magnetresonanzsignals auf wie Antennenspule, rauscharmer Vorverstärker und/oder Analog-DigitalWandler auf.

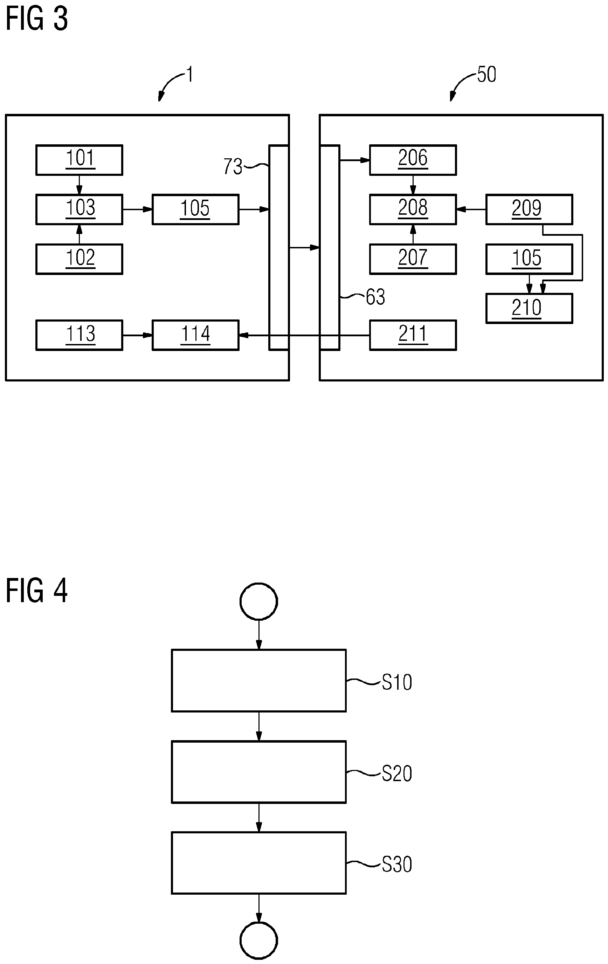

In Fig. 3 ist ein durch die kryptographische Einheit 60 gesicherter Informationsaustausch zwischen dem Peripheriegerät, hier beispielsweise eine Lokalspule 50 und dem Medizingerät, hier ein Magnetresonanztomograph 1, dargestellt. Der Informationsaustausch betrifft eine Challenge-Response-Anfrage.

Bei der Anfrage des Medizingeräts an das Peripheriegerät wird die Anfrage 101 mit einer Hash-Funktion 103 und dem Passwort 102 in einen Hashtag 105 umgesetzt. Als Hash-Funktionen 103, 208 können standardisierte Verfahren angewendet werden wie SHA1,2,3,256, etc, oder andere Verfahren, wie z.B. MD5. Die Anfrage 101 wird zusammen mit dem Hashtag 105 an die kryptographische Einheit 60 der Lokalspule 50 übertragen werden. Die kryptographische Einheit 60 erzeugt ebenfalls aus ihrem lokalen Passwort 207 und der übertragenen Anfrage 206 des Medizingeräts lokal mit der Hash-Funktion 208 einen lokalen Hashtag 209. Nur wenn der lokale Hashtag 209 durch den Komparator 210 als identisch zu dem vom Medizingerät übertragenen Hashtag 105 erkannt wird, wird die Anfrage 101 als vertrauenswürdig angesehen. Das Peripheriegerät antwortet nur dann oder nur dann mit der angefragten Information, wenn sie die Anfrage als vertraulich erkannt hat.

Die Rückantwort des Peripheriegeräts an das Medizingerät läuft in gleicher Weise, jedoch in umgekehrter Richtung ab. Die Antwortinformation wird zusammen mit dem lokalen Passwort 207 mit der lokalen Hash-Funktion 208 in einen lokalen Hashtag 209 überführt. Die Spule antwortet mit der angefragten Antwortinformation und dem lokalen Hashtag 209.

Das Medizingerät erkennt das Peripheriegerät nur als gültig an, wenn ein Vergleich des von dem Peripheriegerät übermittelten lokalen Hashtags 208 zu der übertragenen Antwortinformation passt. Falls dies nicht passt, wird das Peripheriegerät 60 als ungültig erkannt und ein Betrieb des Peripheriegeräts 60 wird von dem Medizingerät ausgeschlossen.

Bei einem Magnetresonanztomographen 1 kann dies durch Ausgabe einer Fehlermeldung und/oder dem Verhindern des Starts einer Messung erreicht werden. Außerdem kann die Tatsache, dass eine solche gefälschte Lokalspule 50 am Magnetresonanztomographen 1 erkannt wurde, an eine zentrale Servicedatenbank weitervermittelt werden, um Missbrauchsversuche aufzuspüren.

Vorzugsweise ist die Kommunikation zwischen Medizingerät und Peripheriegerät durch die Verschlüsselungseinheiten 63, 73 gesichert. Die Authentifizierung durch das Hashtag ist aber unabhängig von den Verschlüsselungseinheiten 63, 73 gesichert und kann auch unverschlüsselt übertragen werden.

Die Identifikation des Peripheriegeräts mit Herstellname, Spulentyp, Revisionsstand und weiteren technischen Merkmalen (Kanalzahl, Geometrie, Feldstärke etc.) und somit die ganze Kommunikation des Systems mit der Lokalspule 50 kann über ein solches Challenge-Response-Verfahren abgewickelt werden.

In einer anderen Implementierung könnend diese Informationen aber auch einfach in Klartext oder nur verschlüsselt ausgetauscht werden. Vorzugsweise wird lediglich die Authentifizierung der Lokalspule dann über ein Challenge-Response-Verfahren abgewickelt.

Zur Identifikation können zusätzlich oder an Stelle des beschriebenen Challenge-Response-Verfahrens auch sehr einfache Merkmale moderner EEPROMs benutzt werden. Moderne EEPROMs enthalten eindeutige und unveränderbare 64-Bit Identifikationsnummern ähnlich zur sog. MAC Adresse in Netzwerkbausteinen, womit jedes EEPROM ein eindeutiges Merkmal besitzt. Zur Authentifizierung eines Peripheriegeräts kann also auch diese ID ausgelesen werden, beispielsweise mit der beschriebenen Challenge-Response, und mit einem ID-Register in einem Speicher des Medizingeräts verglichen werden. Das Medizingerät besitzt dabei Kenntnis über alle möglichen zulässigen EEPROM-IDs oder bestimmte Adress-Bänder. Das Medizingerät kann per SW-Update aus einer zentralen Datenbank, auch in einer Cloud, neue Adressen beziehen. Vorzugsweise tut es dies regelmäßig, sodass auf installierten Medizingeräten auch neue Peripheriegeräte immer funktionsfähig bleiben. Dies ermöglicht auch das Sperren von bestimmten Peripheriegeräten, z.B. falls Sicherheitsprobleme vom Hersteller erkannt wurden, indem die zugehörigen IDs aus dem Register der zulässigen IDs gelöscht werden.

Fig. 4 zeigt eine schematische Darstellung eines Ablaufplans einer Ausführungsform des erfindungsgemäßen Verfahrens.

Das erfindungsgemäße Verfahren ist zum Betrieb eines Medizingeräts mit einem erfindungsgemäßen Peripheriegerät vorgesehen, insbesondere für einen Magnetresonanztomographen 1 und einer bereits beschriebenen Lokalspule 50.

Das Verfahren weist den Schritt S10 des Authentifizierens des Peripheriegeräts durch das Medizingerät mittels einer Kommunikation mit der kryptografischen Einheit 60 auf.

Das Authentifizieren kann beispielsweise durch ein Challenge-Response-Verfahren erfolgen, wie es zu Fig. 3 bereits erläutert wurde.

Denkbar sind aber je nach Sicherheitsanforderung auch einfachere Verfahren, beispielsweise die Übermittlung einer durch die kryptographische Einheit 60 gesicherten Übertragung einer Information von der Lokalspule 50 zu dem Magnetresonanztomographen, z.B. mittels Verschlüsselung zwischen den Verschlüsselungseinheiten 63, 73. Anschließend vergleicht die Steuerung 23 des Magnetresonanztomographen 1 die übertragene Information mit in einem Speicher oder einer Datenbank gespeicherten Werten zulässiger Informationen. Die Information kann Herstellname, Spulentyp, Revisionsstand und weiteren technischen Merkmale, oder auch Seriennummern der Spule, einer Schnittstelle (MAC Adresse) eines Speicherbausteins umfassen. Modernen EEPROMs enthalten eindeutige und unveränderbare 64-Bit Identifikationsnummern (IDs), womit jedes EEPROM ein eindeutiges Merkmal besitzt. Eine Authentifizierung ist in diesem Fall erfolgreich, wenn die übermittelte Information mit einem als zulässig identifizierten gespeicherten Wert übereinstimmt.

In einem Schritt S20 erfolgt nach erfolgreicher Authentifizierung eine Freigabe einer Nutzung des Peripheriegeräts durch das Medizingerät, beispielsweise werden einer, mehrere oder alle notwendigen Schritte für die Bilderfassung von der Steuerung 23 veranlasst und nacheinander ausgeführt. Bei nicht erfolgreicher Authentifizierung wird hingegen mindestens eine notwendige Funktion durch die Steuerung bei erfolgloser Authentifizierung blockiert, d.h. die Ausführung durch die Steuerung 23 verhindert. Das Blockieren kann auch eine Ausgabe einer Information in Schritt S30 an einen Nutzer umfassen.

In einer denkbaren Ausführungsform des erfindungsgemäßen Verfahrens weist die kryptographische Einheit 60 einen gesicherten Zähler auf. Der gesicherte Zähler kann im Betrieb durch einen Vorgang nur auf eine Art verändert werden, beispielsweise entweder nur dekrementiert oder nur inkrementiert werden. Ein Zurücksetzen ist gar nicht oder nur durch einen kryptographisch gesicherten Vorgang möglich, wie er zu Fig. 3 dargestellt ist. Dies ermöglicht z.B. Servicepersonal, den Zähler nach einer Wartung zurückzusetzen.

Der kryptographisch gesicherte Zähler kann beispielsweise ein Datum in dem kryptographisch gesicherten Speicher 62 sein, der von dem Prozessor 61 der kryptographischen Einheit 60 manipuliert wird. Beispielsweise kann bei jedem Stecken des Peripheriegeräts der Zähler um den Wert eins reduziert werden.

Das Authentifizieren in Schritt S10 erfolgt in dieser Ausführungsform in Abhängigkeit von einem vorbestimmten Zählerstand des kryptographisch geschützten Zählers. Beispielsweise kann die übertragene Information der Wert des Zählers sein. Eine erfolgreiche Authentifizierung kann es dabei sein, wenn der Wert des Zählers nicht einen vorbestimmten Wert unterschreitet (bei Dekrementieren) oder überschreitet (bei Inkrementieren). So kann die Nutzung des Peripheriegeräts auf eine vorbestimmte, sichere Anzahl von Nutzungen begrenzt werden. Die Anzahl der Nutzungen kann auch entsprechen bei Einweggeräten gleich eins sein. Die Authentifizierung kann auch die logische Verknüpfung von Vergleichsoperationen für mehrere übertragene Informationen sein wie beispielsweise eine ID und ein Zähler.

Um zu verhindern, dass beispielsweise bei einem Einstecken des Peripheriegeräts in einen für die vorgesehene Anwendung falschen Steckplatz diese nach einem Stecken in einen korrekten Steckplatz das Peripheriegerät nicht mehr ordnungsgemäß genutzt werden kann, ist es denkbar, dass die Authentifizierung für das gleiche Peripheriegerät, z.B. erkennbar am ID, auch bei einem abgelaufenen Zähler, für eine vorbestimmte Zeit und/oder vor einer anderen Aktion wie das Durchführen einer ordnungsgemäßen Nutzung des Peripheriegeräts erfolgreich ist. So kann verhindert werden, dass z.B. nach einem versehentlichen Stecken an einem falschen Steckplatz ein erfindungsgemäßes Einweg-Peripheriegerät verworfen werden muss, weil die Nutzung verweigert wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Peripheriegerät für ein Medizingerät, wobei das Peripheriegerät eine kryptographische Einheit (60) für eine Kommunikation mit dem Medizingerät aufweist.

2. Peripheriegerät nach Anspruch 1, wobei das Medizingerät ein Magnetresonanztomograph (1) ist.

3. Peripheriegerät nach Anspruch 2, wobei das Peripheriegerät eine Lokalspule (2) ist.

4. Peripheriegerät nach einem der vorhergehenden Ansprüche, wobei die kryptografische Einheit (60) ausgelegt ist, die Kommunikation des Peripheriegeräts mit dem Medizingerät zu verschlüsseln.

5. Peripheriegerät nach einem der vorhergehenden Ansprüche, wobei die kryptographische Einheit (60) ausgelegt ist, ein kryptographisches Geheimnis zu speichern und in der Kommunikation mit dem Medizingerät eine Challenge-Response zu beantworten.

6. Peripheriegerät nach einem der vorhergehenden Ansprüche, wobei die kryptographische Einheit (60) einen geschützten Zähler aufweist.

7. System aus einem Peripheriegerät nach einem der vorhergehenden Ansprüche und einem Medizingerät, wobei das Medizingerät ausgelegt ist, mit der kryptographischen Einheit (60) zu kommunizieren.

8. Verfahren zum Betrieb eines Medizingeräts und einem Peripheriegerät nach einem der vorhergehenden Ansprüche, wobei das Verfahren die Schritte aufweist:
Authentifizieren (S10) des Peripheriegeräts durch das Medizingerät mittels einer Kommunikation mit der kryptografischen Einheit (60);
Freigabe (S20) einer Nutzung des Peripheriegeräts durch das Medizingerät.

9. Verfahren nach Anspruch 8, wobei die kryptographische Einheit einen kryptographisch geschützten Zähler aufweist, wobei das Authentifizieren in Abhängigkeit von einem vorbestimmten Zählerstand des kryptographisch geschützten Zählers erfolgt.
